## Europäisches Patentamt

## European Patent Office

## Office européen des brevets

(19)

(11) Veröffentlichungsnummer: **0 043 049**
**B1**

(12) **EUROPÄISCHE PATENTSCHRIFT**

(45) Veröffentlichungstag der Patentschrift:
02.03.83

(21) Anmeldenummer: 81104739.8

(22) Anmeldetag: **20.06.81**

(51) Int. Cl.³: **C 07 C 31/02,** C 07 C 29/04,
C 07 C 31/10, C 07 C 31/12

(54) Verfahren zur Herstellung von niederen Alkoholen.

(30) Priorität: **27.06.80 DE 3024146**

(43) Veröffentlichungstag der Anmeldung:
06.01.82 Patentblatt 82/1

(45) Bekanntmachung des Hinweises auf die Patenterteilung:
02.03.83 Patentblatt 83/9

(84) Benannte Vertragsstaaten:
AT BE DE FR GB IT NL SE

(56) Entgegenhaltungen:
DE-A-1 249 845
DE-A-2 147 739
DE-A-2 429 770
CHEMICAL ABSTRACTS, Band 84, Nr. 23, 7. Juni 1976, Seite 412, Nr. 164 150j Columbus, Ohio, U.S.A.

(73) Patentinhaber: **DEUTSCHE TEXACO AKTIENGESELLSCHAFT**, Überseering 40,
D-2000 Hamburg 60 (DE)

(72) Erfinder: **Neier, Wilhelm, Dr.**, An der Landwehr 40,
D-4134 Rheinberg 3 (DE)
Erfinder: **Webers, Werner**, Rektor-Horn-Strasse 42,
D-4134 Rheinberg 3 (DE)
Erfinder: **Ostwald, Wolf Dr.**, Rheinkamper Strasse 35,
D-4134 Rheinberg 3 (DE)

(74) Vertreter: **Schupfner, Gerhard et al, Patentanwälte Dipl.-Ing. Hans-Jürgen Müller Dipl.-Chem.Dr.phil.nat. Gerhard Schupfner Dipl.-Ing. Hans-Peter Gauger,
Karlstrasse 5 D-2110 Buchholz in der Nordheide (DE)**

## Verfahren zur Herstellung von niederen Alkoholen

Die Erfindung betrifft ein Verfahren zur kontinuierlichen Herstellung von niederen Alkoholen, insbesondere von Isopropylalkohol und sek. Butylalkohol, die durch Hydratisierung von Olefinen, insbesondere Propen und n-Buten, in Gegenwart stark saurer Kationenaustauscher als Katalysator in einem als Sumpffestbett ausgeführten Einrohrreaktor mit beliebigem Durchmesser hergestellt werden.

Durch DE-AS 2 429 770 ist bereits ein Verfahren zur kontinuierlichen Herstellung von niederen Alkoholen an stark sauren Kationenaustauschern bekannt geworden, bei dem der gebildete Alkohol dampfförmig zusammen mit dem überschüssigen Reaktionsgas am Kopf des Reaktors abgezogen wird und nach Entfernung des Gasanteils in Form eines über 80%igen Alkohols gewonnen wird. Bei diesem Verfahren werden jedoch Raktionswasser und Reaktionsgas getrennt vorgeheizt bzw. verdampft. Infolge erheblicher Abkühlung des Reaktorbettes während der Reaktion, und zwar bereits schon nach kurzer Reaktionsstrecke (0,8 – 1,2 m), kann diese nur in Einrohr-Reaktoren bis zu einem Durchmesser von etwa 100 mm durchgeführt werden. Großreaktoren werden daher für dieses Verfahren stets als Vielrohr-Reaktoren eingesetzt, wobei sich bei der Sumpffahrweise Probleme der gleichmäßigen Verteilung von Gas- und Flüssigphasen einstellen.

Dieser Abkühlungseffekt im Verlauf der Reaktion konnte nicht erklärt werden. Aus DE-AS 1 493 190 war bekannt, daß Gase unter überkritischen Bedingungen Alkohole aufnehmen und diese durch Entspannung oder Erwärmen unter gleichem Druck wieder gewonnen werden können. In dieser Auslegeschrift Seite 9, Zeilen 10 bis 23 und 53 bis 67, wird festgestellt, daß die Aufnahme und Abgabe dieser Alkohole oder anderer zu keinen wesentlichen Abkühl- oder Aufheizeffekten des Systems führt.

Der vorliegenden Erfindung lag die Aufgabe zugrunde, das Verfahren zur Herstellung von niederen Alkoholen durch Direkthydration von Olefinen in einem als Sumpfbett ausgeführten Einrohrreaktor mit beliebigem Durchmesser so durchzuführen, daß konstante Temperatur- und Druckbedingungen eingehalten werden können.

Es wurde überraschenderweise gefunden, daß die Reaktionsgasgemische unter überkritischen Bedingungen für die Gase, für Propen/Propan und n-Butene/n-Butan insbesondere unter den Reaktionsbedingungen von 135°C bis 145°C und mindestens 60 bar bzw. 155 bis 165°C und mindestens 40 bar, unabhängig von der Beladung der Gase mit dem gebildeten Alkohol jeweils Wasser, 1,4 Gew.-% Wasser bei Propen/Propan und 1,8 Gew.-% Wasser bei n-Buten/Butan aufnehmen. Durch die Aufnahme dieser Wassermenge und die gebildeten Alkohole im Katalysatorbett wird die Temperatur nach kurzer Reaktorstrecke bei Reaktoren mit einem Durchmesser von größer als 150 mm auch bei äußerer Beheizung derart abgesenkt, daß die Reaktion trotz der Bildungswärme von z. B. 37,6 KJ/Mol bei SBA und 50,2 KJ/Mol IPA fast zum Erliegen kommt.

Der Abkühlung des Reaktorbettes kann überraschenderweise dadurch begegnet werden, daß das Olefin vor seiner Einspeisung in dem Reaktor mit Wasser vorgesättigt wird.

Gegenstand der Erfindung ist ein Verfahren nach Anspruch 1. Vorzugsweise wird das Olefin in Gegenwart eines Teils des Reaktionswassers verdampft, wobei dem Olefin vor der Verdampfung 0,3 bis zu 1,8 Gew.-%, vorzugsweise 1,0 bis zu 1,8 Gew.-% zugesetzt werden.

Es wurde gefunden, daß erfindungsgemäß jede beliebige Reaktionstemperatur eingestellt werden kann, ohne daß Temperaturprobleme im Einrohrreaktor auftreten. Es ist sogar möglich, durch Wahl der Wassermenge die gemeinsam mit dem Reaktionsgas, etwa Propan/Propylen oder Butan/Butylen, verdampft wird, den Gesamtprozeß schwach exotherm, praktisch isotherm oder schwach endotherm ablaufen zu lassen.

Das Verfahren der vorliegenden Erfindung hat den Vorteil, daß die bei anderen bekannten Direkthydratationsverfahren auftretenden Schwierigkeiten mit der Abfuhr der Reaktionswärme bzw. Zufuhr von Wärme nicht auftritt und somit im Katalysatorbett eine sehr gleichmäßige Temepratur eingehalten werden kann. Die Bildung von hot spots kann zuverlässig ausgeschlossen werden.

Die Vorsättigung erfolgt in an sich bekannter Weise in Gasverdampfern, wie diese aus dem Stand der Technik bekannt sind. Die Einspeisung des für die gleichzeitige Verdampfung mit dem Oletin vorgesehenen Wasser erfolgt vorzugsweise zusammen mit dem Olefin in den Verdampfer. Für die Verdampfung hat sich ein vierzügiger Rohrbündelwärmeaustauscher mit Kompensator als besonders geeignet erwiesen, der durch dreimaliges Umlenken des Dampfstroms für eine gute Kontaktierung von Wasser und Olefin sorgt. Das Prinzip eines solchen Wärmeaustauschers ist bekannt. Ebenso können auch andere Wärmeaustauschertypen als Verdampfer eingesetzt werden.

### Vergleichsbeispiel 1

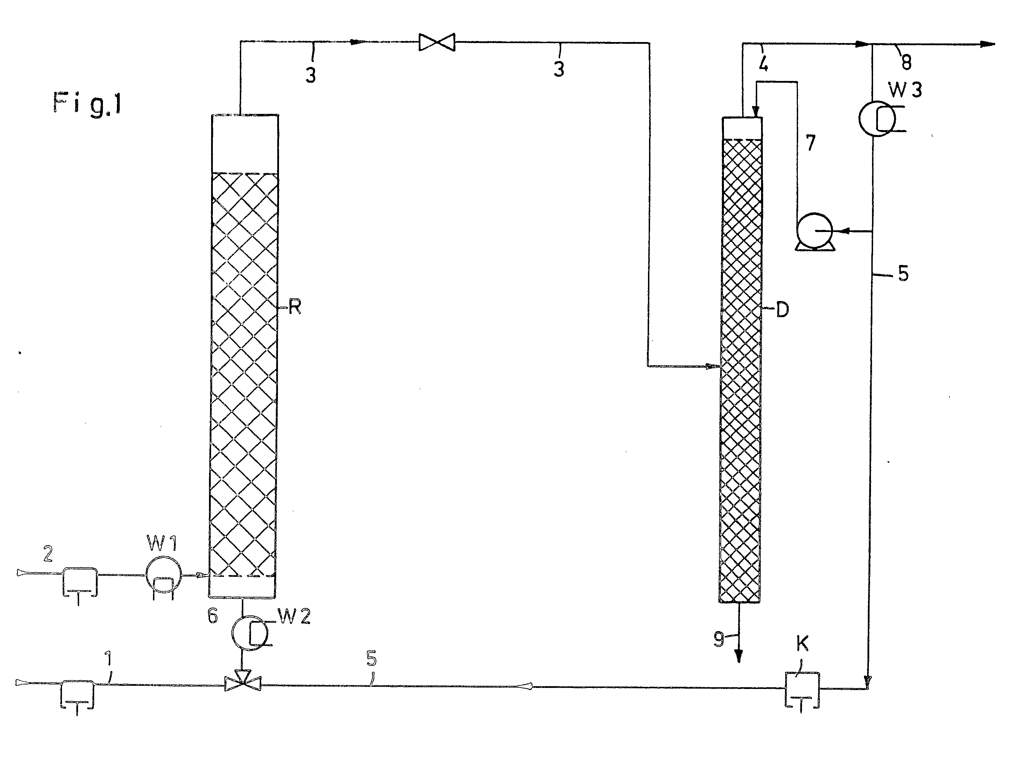

In einem Reaktor von 500 mm lichter Weite und 10 m Länge, der mit 1900 l Edelstahlfüllkörper und mit 1700 l eines stark sauren Ionenaustauschers als Katalysator gefüllt war, wurden über Leitung 1 (siehe Schema, Abb. 1) stündlich 300,3 kg eines 85% n-Buten enthaltenden Butan/Buten-Gemisches (4534 Mole Buten) und über Leitung 2 204,0 kg Wasser (11 333 Mole) eingespeist. Das Wasser wurde im Vorheizer W-1 auf eine Temperatur von 155 bis 160°C vorgeheizt. Das über Leitung 1 dosierte Einsatzgas wurde mit dem über Leitung 5 zurückgeführten Kreislaufgas gemischt und im Verdampfer W-2 verdampft und auf eine Temperatur von 160°C gebracht. Im Reaktor wurde ein Reaktionsdruck von 60 bar aufrechterhalten. Am Kopf des Reaktors wurde der gebildete sek. Butylalkohol zusammen mit dem überschüssigen $C_4$-Gas abgezogen, entspannt, vom n-Butan/n-Buten-Gemisch getrennt und über Leitung 9 abgezogen. Das überschüssige n-Butan/n-Buten-Gemisch wurde zum überwiegenden Teil über Leitung 5 dem Reaktor erneut zugeführt. Über Leitung 8 wurde ein Teilstrom als Restgas ausgeschleust.

Bei dieser Versuchsanordnung wurde nach einer Reaktionsstrecke von 20 cm im Katalysatorbett eine Reaktionstemperatur von 155 bis 158°C gemessen. Trotz exothermer Reaktion (37,6 KJ pro Mol SBA Hydratationswärme) und einer 100 mm starken Isolierung fiel die Reaktionstemperatur nach kurzer Reaktionsstrecke stark ab und erreichte am Reaktorkopf nur noch 115 bis 120°C. Aufgrund des starken Temperaturgefälles im Reaktionsbett wurde lediglich eine mittlere Katalysatorleistung von 0,4 Mol SBA/ 1 Katalysator · h erzielt.

Der im Vergleichsbeispiel beschriebene Versuch wurde mit der Änderung wiederholt, daß der Reaktor jetzt zusätzlich mit einer Rohrschlange versehen war, die mit 9 bar Dampf beaufschlagt war. Trotz Außenwandtemperatur von 160 bis 165°C konnte nur eine geringfügige Verbesserung des Temperaturprofils erreicht werden. Die mittlere Katalysatorleistung betrug 0,45 Mol sek. Butylalkohol/l Kontakt · h.

### Beispiel 1

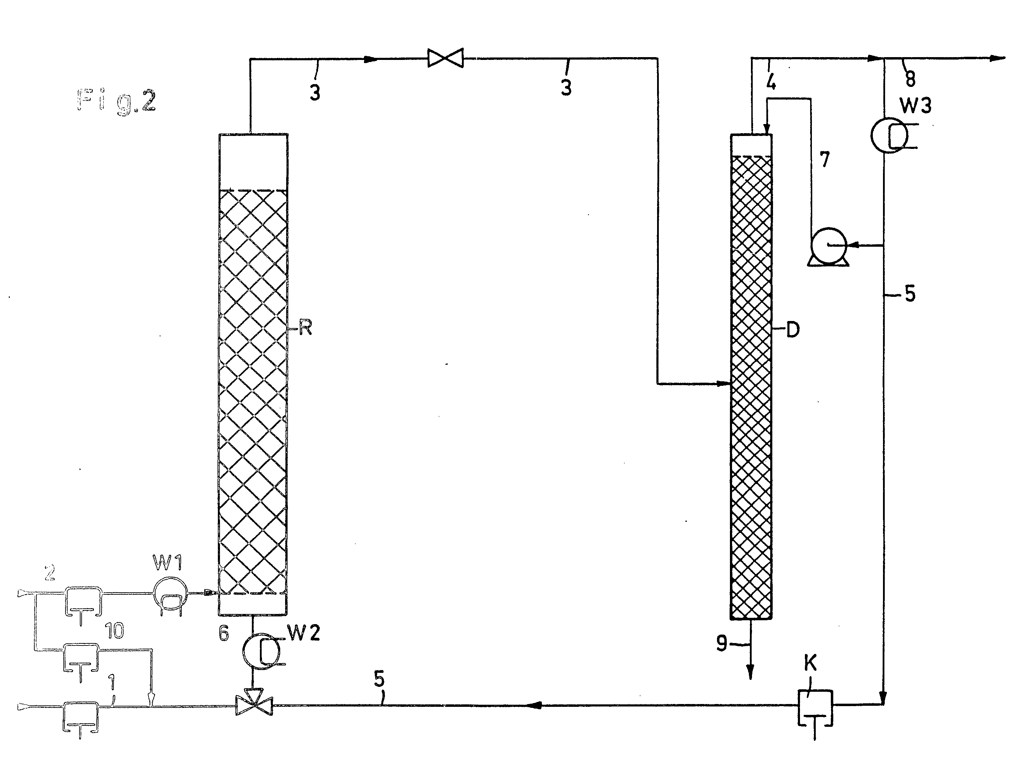

Das im Vergleichsbeispiel 1 beschriebene Verfahren wurde so geändert (Abb. 2), daß mit Hilfe einer weiteren Dosierpumpe 54 kg des Prozeßwassers (von insgesamt 204 kg) über Leitung 10 dem n-Butan/n-Buten-Gemisch (Frischgas + Kreislaufgas = 3000 kg/h) zugemischt und gemeinsam dem Verdampfer W-2 zugeführt werden. Unter den sonst gleichen Bedingungen wie im Vergleichsbeispiel 1 (155 bis 160°C, 70 bar) konnte jetzt im gesamten Reaktor ohne zusätzliche Beheizung oder Kühlung eine Temperatur von 155 bis 162°C aufrechterhalten werden. Am Sumpf der Kolonne D konnten über Leitung 9 stündlich durchschnittlich 201,3 kg (2720 Mol) sek. Butylalkohol und 2,1 kg (16,2 Mol) Di-sek.-Butyläther in Form eines ca. 99%igen Roh-Alkohols gewonnen werden. Über Leitung 8 wurden stündlich 101,5 kg n-Buten und 46,4 kg n-Butan in Form eines ca 59%igen Gemisches abgezogen.

Pro Liter Katalysator wurden stündlich 1,6 Mol sek. Butylalkohol und 0,0095 Mol Äther gebildet.

### Vergleichsbeispiel 2

Die im Vergleichsbeispiel 1 beschriebene Apparatur wird zur Herstellung von Isopropylalkohol eingesetzt. Dazu werden stündlich über Leitung 1 231,3 kg eines 92%igen Propan-/Propen-Gemischs zusammen mit dem über Leitung 5 zurückgeführten Kreislaufgases (2000 kg/h) nach Verdampfung und Erhitzung auf 135°C im Verdampfer W-2 am Sumpf in den Reaktor eingespeist. Über Leitung 2 werden 200 kg Reaktionswasser auf 135°C erhitzt und unten im Reaktor zudosiert. Es wird ein Reaktionsdruck von 100 bar aufrechterhalten.

Am Kopf des Reaktors wurde der gebildete Isopropylalkohol dampfförmig zusammen mit dem überschüssigen Reaktionsgas abgezogen und nach Entspannung auf 20 bar in der Kolonne D vom Kreislaufgas abgetrennt. Nach einer Reaktionsstrecke von 20 cm konnte eine Temperatur von 134 bis 135°C eingehalten werden. Nach einer Reaktionsstrecke von nur 1,2 m war die Temperatur trotz einer Bildungswärme von 50,2 KJ/Mol Isopropylalkohol auf ca. 114°C abgekühlt. Es wurden stündlich pro Liter Katalysator durchschnittlich 0,5 Mol Isopropylalkohol gebildet.

### Beispiel 2

Vergleichsbeispiel 2 wurde mit der Änderung wiederholt, daß jetzt 30,0 kg des Prozeßwassers mit Hilfe einer weiteren Dosierpumpe über Leitung 10 dem Propan/Propen-Gemisch zugemischt und gemeinsam dem Verdampfer W-2 zugeführt werden. Unter sonst gleichen Bedingungen wie Vergleichsbeispiel 2 kann jetzt im gesamten Reaktor eine Reaktionstemperatur von 135 bis 140°C eingehalten werden. Bei einem Gasumsatz von 75%, bezogen auf die zugeführte Frischgasmenge (231,3 kg 92%iges Propen), konnten über Leitung 9 am Sumpf der Kolonne D stündlich 224,4 kg Isopropylalkohol (3740 Mol) und 2,4 kg (23 Mol) Di-isopropyläther in Form eines ca. 80%igen wäßrigen Rohalkohols abgezogen werden. Es wurden stündlich 2,2 Mol Isopropylalkohol pro Liter Katalysator gebildet, die Selektivität lag bei fast 99%.

### Patentansprüche

1. Verfahren zur kontinuierlichen Herstellung von niederen Alkoholen mit 3—5 Kohlenstoffatomen, insbesondere von Isopropanol und sek. Butylalkohol, durch katalytische Hydratation von niederen Olefinen mit 3—5 Kohlenstoffatomen, insbesondere von Proben und n-Buten, in Gegenwart eines stark sauren Kationenaustauschers als Katalysator, der als Festbett in einem Einrohrreaktor angeordnet und von den Reaktionskomponenten bei einer Temperatur von etwa 120 bis 180°C unter einem Druck von etwa 40 bis 200 bar von unten nach oben durchströmt wird, wobei das Molverhältnis der zugeführten Komponenten Wasser zu Olefin etwa 0,5 bis 10 und insbesondere 1,0 bis 3,0 : 1 beträgt, dadurch gekennzeichnet, daß das Olefin vor seiner Einspeisung in den Reaktor mit 0,3 bis 1,8 Gew.-% Wasser bezogen auf das eingesetzte Olefin vorgesättigt wird.

2. Verfahren nach Anspruch 1, dadurch gekennzeichnet, daß das Olefin mit 1,0 bis 1,8 Gew.-% Wasser vorgesättigt wird.

3. Verfahren nach Anspruch 2, dadurch gekennzeichnet, daß das Olefin in Gegenwart von Wasser verdampft wird.

## Claims

1. Process for continuously producing lower alcohols having 3 to 5 carbon atoms, particularly isopropanol and secondary butyl alcohol, by catalytic hydration of lower olefins having 3 to 5 carbon atoms, particularly propene and n-butene, in the presence of a strongly acidic cation exchange resin catalyst arranged as a fixed bed in a single-tube reactor and flown through upwardly by the reactants at a temperature of approx. 120°C to 180°C and a pressure of approx. 40 to 200 bar, the water to olefin mole ratio being approx. 0.5 to 10.0, particularly 1.0 to 3.0, characterized in that the olefin is presaturated with 0.3 to 1.8 weight-% water based on the olefin charged prior to feeding it to the reactor.

2. Process according to claim 1, characterized in that the olefin is presaturated with 1.0 to 1.8 weight-% water.

3. Process according to claim 2, characterized in that the olefin is evaporated in the presence of water.

## Revendications

1. Procédé de production continue d'alcools inférieurs ayant 3 à 5 atomes de carbone, notamment d'isopropanol et d'alcool sec.-butylique, par hydratation catalytique d'oléfines inférieures ayant 3 à 5 atomes de carbone, notamment de propène et de n-butène, en présence d'un échangeur cationique fortement acide comme catalyseur, qui est disposé en lit fixe dans un réacteur monotubulaire et qui est traversé de bas en haut par les composants réactionnels à une température d'environ 120 à 180°C sous une pression d'environ 40 à 200 bars, le rapport molaire eau : oléfine des composants introduits étant d'environ 0,5 à 10 et notamment de 1,0 à 3,0 : 1, caractérisé en ce que l'oléfine, avant son injection dans le réacteur, est pré-saturée avec 0,3 à 1,8% en poids d'eau par rapport à l'oléfine utilisée.

2. Procédé suivant la revendication 1, caractérisé en ce que l'oléfine est pré-saturée avec 1,0 à 1,8% en poids d'eau.

3. Procédé suivant la revendication 2, caractérisé en ce que l'oléfine est vaporisée en présence d'eau.

Fi g.1

Fig.2